# EUROPEAN PATENT SPECIFICATION

(11) **EP 3 225 207 B1**
(45) Date of publication and mention of the grant of the patent: **01.05.2019**
(21) Application number: 17163774.7
(22) Date of filing: 30.03.2017
(51) Int. Cl.: A61B 34/30, A61B 90/50, A61B 17/34, A61B 17/00, A61B 17/16, A61B 17/29, A61B 18/14, A61B 90/00

(54) **SURGICAL ROBOT/INSTRUMENT SYSTEM**
CHIRURGISCHES ROBOTER-/INSTRUMENTENSYSTEM
SYSTÈME DE ROBOT/D'INSTRUMENT CHIRURGICAL

(30) Priority: 31.03.2016 DE 102016105907
(43) Date of publication of application: 04.10.2017
(73) Proprietor: Tuebingen Scientific Medical GmbH, 72074 Tuebingen (DE)
(72) Inventor: Braun, Marcus, 71093 Weil im Schönbuch (DE)
(74) Representative: Winter, Brandl, Fürniss, Hübner, Röss, Kaiser, Polte - Partnerschaft mbB

(56) References cited:
- EP-A1- 1 334 700
- DE-A1-102012 025 101
- DE-A1-102013 005 982
- US-A- 5 855 583

## Description

The present invention relates to a surgical robot/instrument system as well as to a surgical instrument adapted for being connected to a robot.

### Background of the invention

In modern surgery, an increasing number of surgical interventions are carried out preferably in the form of minimally invasive operations by means of or with the aid of surgery robots which are able to move surgical instruments to their destination in a precise manner and so as to be largely free of any vibrations and initiate corresponding movements of the surgical instrument at this place. As sterility is of top priority in a surgical intervention, the robot/instrument system is subdivided so to speak in a non-sterile and a sterile zone/portion, which are separated from each other by a sterility barrier in the form of a plastic foil, for example. In such arrangement, the major part of the robotic system and the drives are situated in the non-sterile zone, whereas at least the surgical instrument as well as its mounting are arranged in the sterile zone. For moving and actuating the surgical instrument, power transmission trains such as gear units, cable pulls/chain hoists or pressure lines usually extend from the respective drives in the non-sterile zone through the barrier to the surgical instrument and/or its mounting in the sterile zone; for this purpose, suitable ports are provided in the barrier.

In particular in the minimally invasive surgery, the drives for the robotic system have the task to drive and move the surgical instruments or also optical endoscopes in all their motion-related degrees of freedom which are required for the respective operation. This is effected today primarily via a guiding arm (e.g. kinetic system with parallel organs) at whose free end the surgical instrument is firmly mounted but so as to be changeable. Said arm transfers all movements which are required for the instrument fastened to it via a corresponding interface which is mounted or can be mounted on the instrument support. This interface has to have a suitable design, so that the sterility of the instrument is ensured.

Further, the guiding arm moves the instrument e.g. in circular paths around a pivot point which is ideally congruent with an invariant point which is determined by a trocar in the abdominal wall. As a standard, also the movement of the instrument in the axial direction parallel to the trocar is effected by the guiding arm of the robotic system.

### Prior art

It is known to arrange a sort of mechanical interface for the mechanical, electrical and/or hydraulic-pneumatic coupling of a mounting of a surgical instrument on a free, distal end of a robot arm, said interface being configured such that a sterility barrier in the form of a foil can be placed between the interface and the instrument support. To this end, the foil-like barrier is equipped with a mechanism which is formed such that - upon placing the mounting on the interface - the respective power transmission trains on the part of the mounting can reach through the foil-like barrier in order to connect to corresponding connectors on the part of the interface and in this way couple the robotic system-internal power transmission trains to the mounting-internal power transmission trains.

During use of a robot/instrument system with such a design, any movements of the robotic system, of the instrument mount and also of the instrument itself are carried out or effected by means of corresponding drives on the part of the robotic system whose drive outputs are partially in operative connection with the respective actuators on the instrument mount as well as the movement functions on the surgical instrument via the respective power transmission trains.

The previously described concept basically allows to implement the surgical instrument as a so-called "disposable" instrument, i.e. an instrument for one-time use. However, it has turned out that especially the instrument mount is required to have a comparably complicated inner structure, so that it is usually realized as a reusable component whose cleaning and sterilization is elaborate and hence costly.

State of the art can be found in DE 10 2013 005 982 A1 which discloses a medical roboter with an arm comprising a kinematic system with i.e. seven actuated joints for moving a surgical instrument coupled to the arm via an instrument flange and an additional tubus flange, wherein both flanges are moved in accordance to each other due to which the complexity of the kinematic system is necessary.

Further state of the art can be found in EP 1 334 700 A1 which discloses surgery support robot having an instrument with an integrated drive for actuating the instrument tip, wherein the drive is coupled to another drive and link system for axial and swiveling movement of the instrument.

DE 10 2012 025 101 A1 shows a positioning device comprising a robotic arm holding a trocar in which a surgical instrument is received, the trocar having additional robotic arms for swiveling trocar sections relatively to each other in order to generate a swiveling movement of the instrument and also comprising a complex cable-pull driving system for moving the instrument within the trocar in an instrument-longitudinal direction.

### Brief description of the invention

In view of the problems described above, it is the object of the present invention to provide a surgical robot/instrument system or apparatus which facilitates the provision of a sterility barrier. Advantageously, the novel surgical robot/instrument system or apparatus is also supposed to improve the guarantee of sterility.

The above object is achieved by a robot/instrument system or apparatus comprising the features of claim 1. Advantageous configurations of the invention are subject-matter of the dependent claims.

In the following description, in particular the following terms are used which are defined in advance:

| | |
|---|---|
| "Actively driven" | According to the invention, this is to be understood as an internal drive which is associated to the element/unit driven in each case and is preferably directly installed on/in it. |
| "Passively driven" | According to the invention, this is to be understood as an external drive which in fact is associated to the element/unit driven in each case, but is preferably directly installed on/in the robotic system and operatively connected to the element/unit driven in each case via a driving force transmission train in the robotic system. |
| "Trocar" | According to the invention, this is to be understood as a trocar sleeve or also as an endoscope shaft providing a minimally invasive access to a patient for a surgical instrument, for instance through the abdominal wall of a patient. |
| "Instrument tip" | This relates to the distal portion of a surgical instrument preferably of the shaft-like type, said distal portion being provided and designed for surgical tissue manipulations such as gripping, cutting, drilling/milling or the like. |
| "Effector" | This is to be understood as a jaw part or holding piece arranged in/on the instrument tip and intended for instance for a tissue engaging element, which is supported so as to be rotatable around its longitudinal axis relative to an instrument shaft. |
| "Actuation" | This is to be understood as exercising an effector function such as opening/closing a jaw part, for instance its jaw part branches, or advancing/retracting a blade, loop or electrode, etc. on the instrument tip. |
| "Interface" | This relates to the coupling point between the robotic system/holding arm and the surgical instrument/trocar or its mounting. In this context, the coupling point is designed for a preferably detachable mechanical and possibly pneumatic/hydraulic and possibly electrical connection. |

The gist of the present disclosure consists essentially in that the drives required for moving/actuating and/or positioning a surgical instrument - which can be coupled to a robotic system in an exchangeable manner - in the degrees of freedom provided for it (as a rule 6 degrees of freedom + 1 instrument-internal actuation movement) are divided to different units of the robot/instrument system or apparatus, which are already constituent parts of a conventional operation setup.

As a consequence, the drives of the surgical instrument or of the functions thereof (preferably a rotation of the effector, an inclination of the instrument tip supporting the effector, a rotation of the shaft, an actuation of jaw part/effector) - corresponding preferably to 3 degrees of freedom + 1 actuation - are provided in/on the changeable instrument itself, so that it is an "actively driven" surgical instrument, so to speak.

A further degree of freedom is preferably realized by the trocar, further preferred in the form of an "actively driven trocar" comprising a trocar-internal drive (1 degree of freedom). As an alternative, the trocar may also represent a sort of passive instrument unit which is mounted to the robotic system in an exchangeable manner via a suitable interface and whose drive is arranged on the part of the robotic system and coupled to the trocar/its mounting via a force transmission train.

A trocar is an auxiliary device which is used in minimally invasive surgeries in order to provide an access through the abdominal wall of a patient, as well as to seal off the operation site from the operation environment in a gas-tight manner. The sleeve-shaped design provides the access for the rod-shaped surgical instruments which are inserted into the body in this way.

In the context of this invention of an overall system for the robotic surgery, a trocar of this type having the known attributes is expanded such that it fulfils the task of a drive (translation of the instrument shaft parallel to the axis of the trocar) in addition to its existing tasks of providing an access to a patient with a predefined access width.

Preferably, the gear train/the drive for moving the instrument/instrument shaft is to be arranged in the trocar and delivered in a sterile condition; more preferably, it is not the motor itself which delivers the energy for the movement. It is preferred that the drive or motor for the mentioned additional trocar function is in the holding arm/cantilever of the robotic system.

The remaining further 2 degrees of freedom relate to the swiveling of the surgical instrument/trocar by the "actively driven holding arm" of the robotic system preferably around an invariant point (i.e. the translatory movement of the interface in the X- and Y-direction of a preferably horizontal plane), said point being determined by the trocar or the surgical instrument in the abdominal wall of a patient.

To be more precise, the "active" holding arm/cantilever exclusively performs the movement (pivoting) of the surgical instrument/trocar, by the correspondingly driven holding arm/cantilever traveling along a circle segment so to speak, whereas with prior robotic solutions of this type the entire instrument movement has been effected by a robotic kinematic system fastened to the surgical instrument via an adapter.

According to the invention, this is at least partially achieved by the holding arm extending in an essentially horizontal plane or being inclined relative thereto and the interface (gripping or holding device) being extendable and retractable in a telescopic manner relative to the holding arm, or the holding arm being extendable and retractable in the longitudinal direction of the holding arm in a telescopic manner, for moving the instrument shaft transverse to the shaft direction.

With a trocar assembly, the interface is provided on the robotic system/holding arm and designed to not hold and guide the surgical instrument itself, but to hold and guide the trocar (for instance an actively driven trocar or one whose drive is arranged on the part of the robotic system e.g. in the holding arm of the robotic system) into which the surgical instrument is inserted and retained therein.

Here, the holding arm fulfils exclusively the pivoting movement of the trocar around the natural support point, preferably the abdominal wall. Accordingly, also the natural invariant point is used, which results from the placement of the trocar in the abdominal wall.

The active instrument is thus put freely in the (active) trocar, so to speak, which for its part moves the instrument back and forth in relation to the instrument's axis. This is preferably possible if the implemented drives in the active instrument have a compact construction and a low weight, so that the reaction forces acting on the abdominal wall can be neglected in the zone of the support site.

As an alternative or in addition, the holding arm may be configured such that an additional arm supports the trocar in the vicinity of the entry point, for example by a membrane support, in this way quasi simulating the abutment so far defined by the abdominal wall for the definition of the invariant point. As a further alternative, however, it is also conceivable to move the trocar/the surgical instrument in a robotic fashion not only on a circular path (and to effect the pivoting movement by the abdominal wall/the membrane support), but to rotate the gripper simultaneously to the holding arm's movement in order to actively produce the pivoting movement of the trocar/surgical instrument (without the abdominal wall/membrane as an abutment).

In the prior art, the structure of a commonly known surgical instrument for a robot-assisted surgery is characterized in that the drive of such an instrument has to go through the initially mentioned sterile barrier (passive instrument), where there are various solutions. By way of example, it is known to transmit rotational movements via an adapter plate to the surgical instrument, said plate as a transmitter being fastened to a sterile protective foil and hence separating the non-sterile robotic arm from the sterile instrument. This is necessary as the instrument might have to be changed during the operation.

Other systems make use of a so-called port system (as already mentioned at the outset) for providing a connection to the instrument drives, or the elasticity of a membrane situated between the drives or even a simple sterile foil is made use of in a known manner.

The present invention, however, makes provision to essentially do without a motorized or transmission-based connection to the robotic arm for the purpose of driving the surgical (exchangeable) instrument, because the motors (drives) required for driving the surgical instrument are already integrated or installed in the instrument realized as a one-way product. This offers the possibility to basically do without a sterile port for the drives, because the surgical (exchangeable) instrument taken by itself is already sterilized before use, with this condition remaining unchanged even if a corresponding instrument is exchanged.

The drives are controlled preferably in a pneumatic or hydraulic manner. These are, for instance, the drives for those movements which can be directly associated to the instrument, preferably
a) an actuation of the effector (e.g. opening/closing an effector jaw part),
b) an inclination/a bending of the effector for instance in the area of its connection joint to the instrument shaft,
c) a rotation of the instrument tip or of the effector supported therein (around the longitudinal axis of the instrument shaft) and/or
d) a rotation of the instrument shaft itself.

Consequently, all the movements mentioned above are preferably two translatory movement transfers and two rotary movement transfers.

The pneumatic/hydraulic system allows to achieve high forces in small installation spaces in conjunction with low weight. The low weight increases the safety due to a smaller moving load, smaller working spaces of the kinematic holding system and hence follows the philosophy "safety by design". The risk of injury of a user/patient due to a collision between the moved arms and the staff, as known from medical robotics or also industrial robotics, is significantly smaller owing to the claimed invention. What is more, pneumatic or hydraulic drives have a simple structure and thus can result in a product which is intended for a one-time use, if applicable. As an alternative, however, it is also possible to provide electric actuators such as electric motors or piezo elements. It would be advantageous to place the drives (directly) associated to the surgical instrument on the sterile side in such a manner on/in the surgical instrument that its manipulation on/in the patient is not impaired. To this end, provision is made to arrange the drives on a proximal end of the surgical instrument, preferably with respect to an articulation point for the instrument mount. In this case, the actively driven instrument remains essentially unchanged in its distal zone, preferably in the instrument portion between the articulation point for the instrument mount and the distal instrument end, compared to a conventional, passively driven instrument. Thus, the manipulation of the actively driven instrument is not affected as compared to a passively driven instrument and the sight on the surgery or intervention site of the actively driven instrument on the patient body remains unobstructed.

Further advantageous configurations of the invention are, among other things, subject-matter of the dependent claims.

### Description of the Figures

The invention will be explained in more detail below on the basis of a preferred exemplary embodiment with reference to the attached drawings.
Fig. 1 shows the distal portion of a surgical instrument realized with a minimally invasive design, such as it is used, among other things, in a surgical robot/instrument system, for explaining the required degrees of freedom,
Fig. 2 shows the basic design of a surgical robot/instrument system according to a preferred exemplary embodiment of the present invention,
Fig. 3 shows a possible surgery setup, and
Fig. 4 shows the exterior structure of the surgical instrument according to Fig. 1.

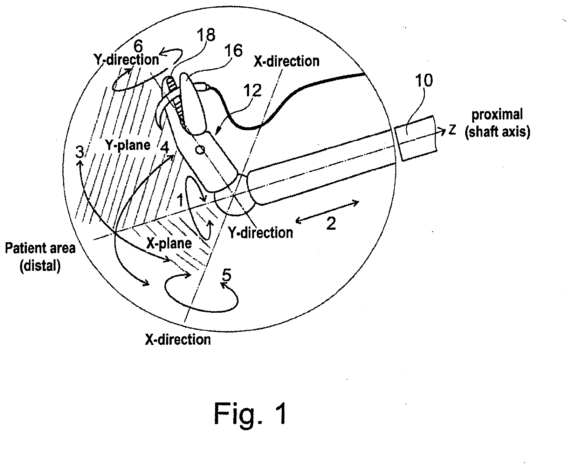

The surgical instrument shown in Fig. 1 which has a minimally invasive design and is formed/adapted for being used in a surgical robot/instrument system according to the preferred exemplary embodiment of the present invention, is realized in this exemplary case as an instrument in the nature of gripping pliers. However, it may also be designed as a mono- or bipolar HF instrument, a mechanical cutting instrument (knife, milling cutter, drill etc.), a loop instrument or as a surgical instrument of similar type.

In the present example, the surgical instrument having a minimally invasive design comprises an instrument shaft 10 (flexible or rigid) at whose distal end an instrument tip 12 is articulated in such a manner that the instrument tip 12 can bend like a hinge with respect to the shaft axis 10. In the following, this bending function forms according to Fig. 1 the 5th degree of freedom of the surgical instrument. Moreover, the instrument shaft 10 is held or supported such that it can rotate around its longitudinal axis (in the following, this corresponds to the 1st degree of freedom of the surgical instrument) and can translatorily move (shift) along its shaft axis, in the following corresponding to the 2nd degree of freedom of the surgical instrument.

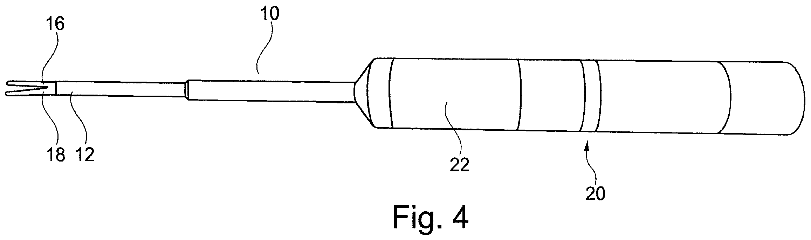

As shown in Fig. 4, the rotational movement of the instrument shaft 10 can be achieved for instance in that the instrument shaft has a two-part design, comprising a distal (separate) shaft portion which is rotatably supported in/on/around a proximal shaft portion, so that the distal shaft portion can be rotated relative to the proximal shaft portion around the longitudinal axis of the (entire) instrument shaft.

Further, provision may be made that the distal shaft portion, in addition to or as an alternative to the previously described rotational support, is supported in/around/on the proximal shaft portion even so as to be movable in the longitudinal direction (in a telescopic manner).

Furthermore, the instrument shaft 10 can be inclined/tilted in an X-plane as well as in a Y-plane (perpendicular to the X-plane), in the following corresponding to the 3rd and 4th degree of freedom of the surgical instrument. Finally, the instrument tip 12 forms or comprises an effector of the surgical instrument, in the present case consisting of a jaw part comprising preferably two branches 16, 18 from which at least one branch 16 is pivotally supported on the effector in order to enlarge or reduce a gripping/clamping gap between the branches. This pivoting movement of the at least one branch 16 represents in the following the 6th degree of freedom of the surgical instrument.

Here, it is referred to the fact that in the case of a surgical instrument realized in some other design, such as a mechanical cutting knife, for example, the 6th degree of freedom would relate to extending or retracting the knife from or into the instrument tip, or in the case of a drill/milling cutter would relate to the rotation of the milling/drilling head, etc. It would also be conceivable to move an HF electrode or similar tool with respect to the effector.

The 5th and 6th degrees of freedom or movement possibilities defined in this way are achieved in the present exemplary embodiment preferably by mechanical ways and means, in fact preferably by means of independent power transmission trains (not shown in further detail) which may be arranged as instrument-internal trains within the instrument shaft 10.

As illustrated in Fig. 4, the surgical instrument comprises one or more drive units 20 (e.g. electric, hydraulic and/or pneumatic motors) which are connected to the instrument shaft 10 at the proximal end (end portion) thereof and to which the instrument-internal power transmission trains for the powered effectuation of the 5th and 6th degree of freedom and optionally the 1st and 2nd degree of freedom of the instrument are coupled or can be coupled. The instrument-internal drive unit(s) is/are each arranged in one housing or a shared housing 22 and may be encapsulated. The housing(s) 22 is/are connected to the instrument shaft 10 (proximal shaft portion) in a fixed or detachable manner. In the first case, the drive unit(s) together with the instrument shaft 10 as well as the instrument tip/effector 12 articulated thereon are configured as a disposable instrument, whereas in the second case only the instrument shaft 10 complete with the instrument tip 12 is disposed after completing the surgery, the instrument-internal drive unit(s) being uncoupled and then reused after a corresponding cleaning/sterilization process.

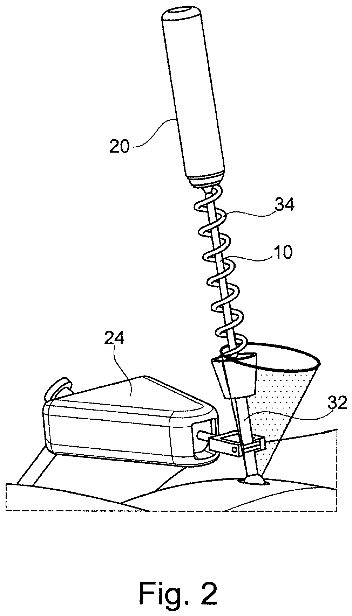

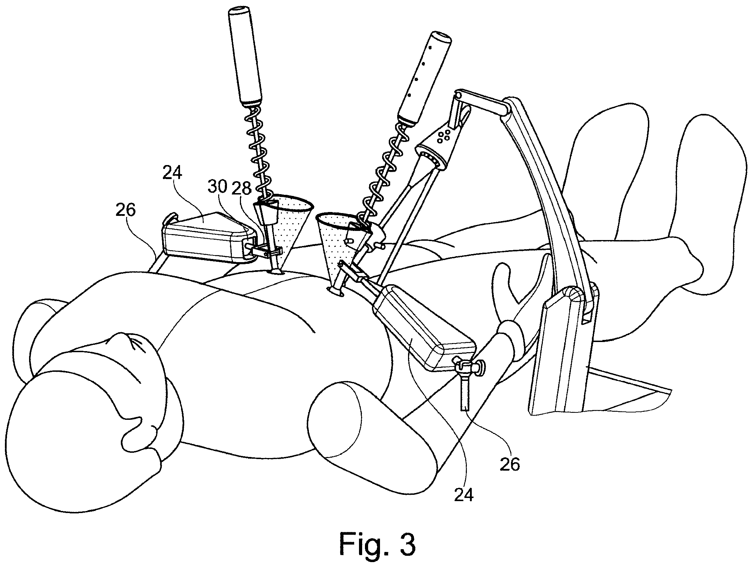

Fig. 2 shows the basic structure of a surgical robot/instrument system or apparatus. Accordingly, the robotic system or robotic structure comprises a holding/cantilever arm 24 which is mounted or supported on a stand 26 indicated in Fig. 3 preferably so as to be vertically adjustable/movable. In this arrangement, the holding arm 24 extends in an essentially horizontal plane, but it may also be aligned so as to be inclined relative thereto. The holding arm 24 is either movable along the stand 26 or additionally extendable (in a telescopic manner) with respect to the stand axis (angularly relative to the stand) preferably in the longitudinal direction of the arm. In addition, there is the option to swivel or rotate the holding arm 26 around the stand axis.

Here, it is referred to the fact that the stand 26 may be immovably mounted or in turn may be arranged on the distal end of a further, preceding movement mechanism (and hence in a movable manner). In the latter case, the stand 26 may simply be a swivel pin where the holding arm 24 is pivotally or immovably supported/held.

It may also be provided to support the holding arm on the stand only so as to be able to pivot, but not so as to be able to extend in telescopic manner, with the option that the holding arm can also be extended in a telescopic manner.

According to the present preferred exemplary embodiment, the holding arm 24 has its distal end portion provided with a gripper or coupling piece 28 which is connected to the holding arm 24 preferably by means of a joint or hinge 30. It is preferred that the gripper 28 can be exchanged depending on the surgical instrument to be used in each case or is designed as a universal gripper (and hence in a not exchangeable manner) which is adapted to be coupled to a freely selected surgical instrument.

The robotic structure is thus designed such that it is capable of moving the gripper 28 arranged on the distal end of the holding arm 24 according to the previously mentioned 3rd and 4th degree of freedom.

Stated in other words, in the case of application of the surgical robot/instrument system it is understood that the surgical instrument is inserted directly or, according to the invention, through a trocar 32 into a patient cavity, e.g. through the abdominal wall. In this case, the patient's tissue (e.g. abdominal wall) representing the penetration site serves as an abutment against any movements in the tissue plane. If the gripper 24 is moved in an X- and Y-direction transverse to the shaft axis, the instrument shaft 10 and/or the trocar 32 perform a corresponding pivoting movement around the penetration site as an imaginary pivot point. In this way, the instrument shaft/trocar may describe a sort of funnel in the course of its pivoting movement, with the penetration site as the tip of the funnel, as indicated in Fig. 2.

As an alternative or in addition to this, the abdominal wall may also be supplemented or replaced by an elastic membrane defining the imaginary pivot point. Finally, it is also possible to give the gripper a corresponding rotation by a motorized unit in order to produce a funnel-shaped pivoting movement of the instrument/trocar in superposition with the circular movement of the holding arm; in this case, the abdominal wall and/or membrane serving as the abutment would not be required any more.

In this connection, the drive unit(s) 20 is/are located with respect to the gripper 24 on an end side of the surgical instrument remote from the penetration site, so that the view onto the penetration site remains unobstructed and is slightly limited merely by the preferably filigree gripper 24 (which is constructed as a framework).

In the embodiments according to the invention, it is not the surgical instrument itself which is mounted on the gripper 24, but a trocar 32 is exchangeably mounted.

A trocar is a surgical introduction aid at least comprising a tubular shaft having a distal front edge preferably realized as a blade and an insertion funnel on the proximal end of the tubular shaft for the insertion of a surgical instrument having the previously mentioned structure.

As a general rule, the trocar is realized so as to have a smooth surface at the inner side of the shaft, preferably with a sealing edge for preventing any uncontrolled outflow of blood or for preventing an air leak in the event of pressurizing the patient cavity with air for deploying it.

In the present exemplary embodiment, however, the trocar 32 is (optionally) provided with an internal drive/drive unit (not shown in further detail) by means of which the inserted surgical instrument can be (optionally) shifted in its longitudinal axis and, if applicable, can also be (optionally) rotated around its longitudinal axis. By way of example, the trocar-internal drive may consist of a number of friction wheels which act on the instrument shaft.

Further, the trocar-internal drive may also be designed such that it acts in one direction only, for instance in a direction toward the patient for advancing the surgical instrument into the patient body, whereas a movement of the surgical instrument in the opposite direction (out of the patient body) can be achieved for instance by a helical compression spring which is supported on the trocar 32 as well as on the drive unit 20 of the surgical instrument, as is likewise shown in Fig. 2.

The operating principle of the surgical robot/instrument system according to the invention can be explained preferably on the basis of Fig. 3:
Here, a patient is illustrated symbolically, which is penetrated at at least two points spaced apart from each other by one trocar in each case. The two trocars are each held on one holding arm 24 (or gripper) in the previously mentioned sense, in fact in such a way that the two trocars are able to perform a pivoting movement (in a funnel shape) around the respective penetration site, as has been described above. In addition, a camera is inserted into the patient body at a third penetration site.

As can be seen in this case, the pivoting movement of each of the trocars is achieved in that the gripper 28 is swiveled back and forth around its joint to the holding arm 24 in a first plane (e.g. X-plane according to Fig. 1), while at the same time the holding arm 24 itself is extended or shortened in a telescopic manner or alternatively the gripper on the holding arm 24 is retracted and extended in a telescopic manner in order to pivot the trocars in a second plane (e.g. Y-plane according to Fig. 1). Moreover, the outer form of the holding arm 24 is clearly visible in Fig. 3, the latter preferably forming a housing for receiving one or more drives designed for actuating the gripper 28 according to the above description.

## Claims

1. A surgical robot/instrument system comprising a trocar (32) which is designed to receive a surgical instrument and a holding arm (24) whose distal end portion has a gripping or holding device (28) arranged thereon, the latter being designed to hold exclusively the trocar (32) in an exchangeable fashion, and the surgical instrument preferably with a minimally invasive design and comprising an instrument shaft (10) whose distal end portion supports an instrument tip (12) via a joint, said instrument tip supporting or forming an effector of the surgical instrument, wherein the surgical robot/instrument system comprises a number of drives or drive units by means of which at least the following functions can be effected:
- an actuation of the effector,
- an inclination or bending of the effector,
- a rotation of the effector around its longitudinal axis and/or a rotation of the instrument shaft (10),
- a movement of the instrument shaft (10) in its shaft direction as well as
- moving the instrument shaft (10) transverse to the shaft direction, wherein
the drives for effecting at least the instrument-internal functions, are provided as instrument-internal drives on/in the surgical instrument, whereas the drives for effecting the instrument-external functions - moving the instrument shaft (10) transverse to the shaft direction are provided as instrument-external drives on/in the holding arm (24); wherein the holding arm (24) extends in an essentially horizontal plane or is inclined relative thereto and
**characterized in that** the gripping or holding device (28) can be extended and retracted relative to the holding arm (24) in a telescopic manner or the holding arm (24) can be extended and retracted in the longitudinal direction of the holding arm (24) in a telescopic manner, for moving the instrument shaft (10) transverse to the shaft direction.

2. The surgical robot/instrument system according to claim 1, **characterized in that** the trocar is designed to receive the surgical instrument so as to be rotatable around its instrument shaft and/or so as to be movable along its instrument shaft.

3. The surgical robot/instrument system according to claim 2, **characterized in that** the trocar is provided with trocar-internal drives which are designed to effect the movements of the surgical instrument within the trocar.

4. The surgical robot/instrument system according to claim 3, **characterized in that** the trocar-internal drives comprise a number of friction wheels which act on the instrument shaft.

5. The surgical robot/instrument system according to claim 4, wherein the friction wheels act on the instrument shaft in one direction only and the movement in the opposite direction is achieved by a helical compression spring.

6. The surgical robot/instrument system according to claim 1, **characterized in that** the gripping or holding device is mounted on the holding arm in such a manner that it can be bent with respect to the holding arm.

7. The surgical robot/instrument system according to claim 1, **characterized in that** the holding arm (24) can be mounted to a stand (26) and can swivel or rotate around a stand axis of the stand (26) for moving the instrument shaft (10) transverse to the shaft direction.

8. The surgical robot/instrument system according to claim 6 or 7, **characterized in that** the bending movement and the telescopic movement are effected by the instrument-external drives.

9. The surgical robot/instrument system according to claim 1, **characterized in that** the instrument-internal drives are connected to the instrument shaft in a fixed or detachable manner and effect the instrument-internal functions via power transmission trains arranged within the instrument shaft.

10. The surgical robot/instrument system according to claim 1, **characterized in that** the instrument-internal functions, comprise
- an actuation of the effector,
- an inclination or bending of the effector at its joint to the instrument shaft (10) and
- a rotation of the effector around its longitudinal axis

## Patentansprüche

1. Chirurgisches Roboter/Instrumentensystem mit einem Trokar (32), der dazu ausgelegt ist, ein chirurgisches Instrument aufzunehmen, und einem Haltearm (24), an dessen distalem Endabschnitt eine Greif- oder Haltevorrichtung (28) angeordnet ist, wobei letztere dazu ausgelegt ist, ausschließlich den Trokar (23) auf austauschbare Weise zu halten, und wobei das chirurgische Instrument vorzugsweise minimalinvasiv ausgelegt ist und einen Instrumentenschaft (10) aufweist, dessen distaler Endabschnitt über ein Gelenk eine Instrumentenspitze (12) hält, welche einen Effektor des chirurgischen Instruments hält oder bildet, wobei das chirurgische Roboter/Instrumentensystem eine Anzahl von Antrieben oder Antriebseinheiten aufweist, durch welche wenigstens die folgenden Funktionen erreicht werden können:
- eine Aktuierung des Effektors,
- ein Neigen oder Abwinkeln des Effektors,
- eine Rotation des Effektors um dessen Längsachse und/oder eine Rotation des Instrumentenschafts (10),
- eine Bewegung des Instrumentenschafts (10) in dessen Schaftrichtung sowie
- ein Bewegen des Instrumentenschafts (10) quer zu der Schaftrichtung,
wobei die Antriebe zum Erreichen wenigstens der instrumenteninternen Funktionen als instrumenteninterne Antriebe an/in dem chirurgischen Instrument bereitgestellt sind, wohingegen die Antriebe zum Erreichen der instrumentenexternen Funktionen
- Bewegen des Instrumentenschafts (10) quer zu der Schaftrichtung als instrumenteninterne Antriebe an/in dem Haltearm (24) bereitgestellt sind,
wobei der Haltearm (24) sich in einer im Wesentlichen horizontalen Ebene erstreckt oder relativ dazu geneigt ist, und
**dadurch gekennzeichnet, dass** die Greif- oder Haltevorrichtung (28) relativ zu dem Haltearm auf teleskopische Weise ausstreckbar und zurückziehbar ist, oder der Haltearm (24) in der Längsrichtung des Haltearms (24) auf teleskopische Weise ausstreckbar und zurückziehbar ist, um den Instrumentenschaft (10) quer zu der Schaftrichtung zu bewegen.

2. Chirurgisches Roboter/Instrumentensystem nach Anspruch 1, **dadurch gekennzeichnet, dass** der Trokar dazu ausgelegt ist, das chirurgische Instrument derart aufzunehmen, dass es um seinen Instrumentenschaft drehbar und/oder entlang seines Instrumentenschafts bewegbar ist.

3. Chirurgisches Roboter/Instrumentensystem nach Anspruch 2, **dadurch gekennzeichnet, dass** der Trokar mit trokarinternen Antrieben versehen ist, welche dazu ausgelegt sind, die Bewegungen des chirurgischen Instruments innerhalb des Trokars zu verursachen.

4. Chirurgisches Roboter/Instrumentensystem nach Anspruch 3, **dadurch gekennzeichnet, dass** die trokarinternen Antriebe eine Anzahl von Reibrädern aufweisen, welche auf den Instrumentenschaft einwirken.

5. Chirurgisches Roboter/Instrumentensystem nach Anspruch 4, wobei die Reibräder nur in einer Richtung auf den Instrumentenschaft einwirken, und die Bewegung in der entgegengesetzten Richtung durch eine Spiraldruckfeder bewirkt wird.

6. Chirurgisches Roboter/Instrumentensystem nach Anspruch 1, **dadurch gekennzeichnet, dass** die Greif- oder Haltevorrichtung an dem Haltearm derart montiert ist, dass sie bezüglich des Haltearms abwinkelbar ist.

7. Chirurgisches Roboter/Instrumentensystem nach Anspruch 1, **dadurch gekennzeichnet, dass** der Haltearm (24) an einem Stand (26) montierbar ist und um eine Standachse des Stands (26) verschwenkbar oder drehbar ist, um den Instrumentenschaft (10) quer zur Schaftrichtung zu bewegen.

8. Chirurgisches Roboter/Instrumentensystem nach Anspruch 6 oder 7, **dadurch gekennzeichnet, dass** die Abwinkelbewegung und die Teleskopbewegung durch die instrumentenexternen Antriebe bewirkt werden.

9. Chirurgisches Roboter/Instrumentensystem nach Anspruch 1, **dadurch gekennzeichnet, dass** die instrumenteninternen Antriebe mit dem Instrumentenschaft auf feste oder abnehmbare Weise verbunden sind und die instrumenteninternen Funktionen über Leistungsübertragungszüge betätigen, die innerhalb des Instrumentenschafts angeordnet sind.

10. Chirurgisches Roboter/Instrumentensystem nach Anspruch 1, **dadurch gekennzeichnet, dass** die instrumenteninternen Funktionen Folgendes aufweisen:
- eine Aktuierung des Effektors,
- ein Neigen oder Abwinkeln des Effektors an dessen Gelenk zu dem Instrumentenschaft (10) und
- eine Rotation des Effektors um seine Längsachse.

## Revendications

1. Système d'instrument/robot chirurgical comprenant un trocart (32) qui est conçu pour recevoir un instrument chirurgical et un bras de maintien (24) dont une portion d'extrémité distale comporte un dispositif de préhension ou de maintien (28) agencé dessus, ce dernier étant conçu pour maintenir exclusivement le trocart (32) de façon échangeable, et l'instrument chirurgical de préférence avec une conception mini-invasive et comprenant une tige d'instrument (10) dont une portion d'extrémité distale supporte une pointe d'instrument (12) via un joint, ladite pointe d'instrument supportant ou formant un organe effecteur de l'instrument chirurgical, dans lequel le système d'instrument/robot chirurgical comprend un certain nombre d'entraînements ou d'unités d'entraînement au moyen desquels au moins les fonctions suivantes peuvent être effectuées :
- un actionnement de l'organe effecteur,
- une inclinaison ou un fléchissement de l'organe effecteur,
- une rotation de l'organe effecteur autour de son axe longitudinal et/ou une rotation de la tige d'instrument (10),
- un déplacement de la tige d'instrument (10) dans sa direction de tige ainsi que
- un déplacement de la tige d'instrument (10) transversalement à la direction de tige, dans lequel
les entraînements pour effectuer au moins les fonctions internes à l'instrument, sont prévus en tant qu'entraînements internes à l'instrument sur/dans l'instrument chirurgical, tandis que les entraînements pour effectuer les fonctions externes à l'instrument, le déplacement de la tige d'instrument (10) transversalement à la direction de tige, sont prévus en tant qu'entraînements externes à l'instrument sur/dans le bras de maintien (24) ;
dans lequel le bras de maintien (24) s'étend dans un plan essentiellement horizontal ou est incliné par rapport à celui-ci et
**caractérisé en ce que**
le dispositif de préhension ou de maintien (28) peut être étendu et rétracté par rapport au bras de maintien (24) de manière télescopique ou le bras de maintien (24) peut être étendu et rétracté dans la direction longitudinale du bras de maintien (24) de manière télescopique, pour déplacer la tige d'instrument (10) transversalement à la direction de tige.

2. Système d'instrument/robot chirurgical selon la revendication 1, **caractérisé en ce que** le trocart est conçu pour recevoir l'instrument chirurgical de façon à pouvoir tourner autour de sa tige d'instrument et/ou de façon à être mobile le long de sa tige d'instrument.

3. Système d'instrument/robot chirurgical selon la revendication 2, **caractérisé en ce que** le trocart est pourvu d'entraînements internes au trocart qui sont conçus pour effectuer les déplacements de l'instrument chirurgical au sein du trocart.

4. Système d'instrument/robot chirurgical selon la revendication 3, **caractérisé en ce que** les entraînements internes au trocart comprennent un certain nombre de roues à friction qui agissent sur la tige d'instrument.

5. Système d'instrument/robot chirurgical selon la revendication 4, dans lequel les roues à friction agissent sur la tige d'instrument dans une direction uniquement et le déplacement dans la direction opposée est obtenu par un ressort de compression hélicoïdal.

6. Système d'instrument/robot chirurgical selon la revendication 1, **caractérisé en ce que** le dispositif de préhension ou de maintien est monté sur le bras de maintien de telle manière qu'il peut être fléchi par rapport au bras de maintien.

7. Système d'instrument/robot chirurgical selon la revendication 1, **caractérisé en ce que** le bras de maintien (24) peut être monté sur un pied (26) et peut pivoter ou tourner autour d'un axe de pied du pied (26) pour déplacer la tige d'instrument (10) transversalement à la direction de tige.

8. Système d'instrument/robot chirurgical selon la revendication 6 ou 7, **caractérisé en ce que** le mouvement de flexion et le mouvement télescopique sont effectués par les entraînements externes à l'instrument.

9. Système d'instrument/robot chirurgical selon la revendication 1, **caractérisé en ce que** les entraînements internes à l'instrument sont raccordés à la tige d'instrument de manière fixe ou amovible et effectuent les fonctions internes à l'instrument via des trains de transmission de puissance agencés au sein de la tige d'instrument.

10. Système d'instrument/robot chirurgical selon la revendication 1, **caractérisé en ce que** les fonctions internes à l'instrument, comprennent
- un actionnement de l'organe effecteur,
- une inclinaison ou une flexion de l'organe effecteur au niveau de son joint avec la tige d'instrument (10) et
- une rotation de l'organe effecteur autour de son axe longitudinal.
